# EUROPEAN PATENT SPECIFICATION

(11) **EP 3 053 439 B1**
(45) Date of publication and mention of the grant of the patent: **17.01.2018**
(21) Application number: 16154905.0
(22) Date of filing: 09.02.2016
(51) Int. Cl.: A01N 25/00, A01N 63/00, A01K 67/033, A23K 10/20, A23K 50/90

(54) **MITE COMPOSITION, METHOD FOR THE APPLICATION OF AT LEAST ONE POPULATION OF MITE SPECIES BELONGING TO THE ASTIGMATA IN ORDER TO PROVIDE AN IN-CROP FOOD SOURCE FOR MITE PREDATORS OF THE PHYTOSEIIDAE, WHEN BEING USED AS BIOLOGICAL CONTROL AGENTS**
MILBENZUSAMMENSETZUNG, VERFAHREN ZUR ANWENDUNG MINDESTENS EINER POPULATION VON ZU DEN ASTIGMATA GEHÖRENDEN MILBEN, UM EINE NAHRUNGSQUELLE AUF DER PFLANZE FÜR MILBENFEINDE DER PHYTOSEIIDAE BEREITZUSTELLEN, WENN SIE ALS BIOLOGISCHE PFLANZENSCHUTZMITTEL VERWENDET WERDEN
COMPOSITION D'ACARIENS, PROCÉDÉ POUR L'APPLICATION D'AU MOINS UNE POPULATION D'ESPÈCES D'ACARIENS APPARTENANT À L'ESPÈCE ASTIGMATA AFIN DE FOURNIR UNE SOURCE ALIMENTAIRE EN CULTURE POUR LES ACARIENS PRÉDATEURS DU PHYTOSEIIDAE, LORSQU'IL EST UTILISÉ EN TANT QU'AGENTS DE LUTTE BIOLOGIQUE

(30) Priority: 09.02.2015 EP 15154397; 11.02.2015 EP 15154668
(43) Date of publication of application: 10.08.2016
(73) Proprietor: Agrobio S.L., 04745 El Viso (La Mojonera) - Almeria (ES)
(72) Inventor: Griffiths, Donald Alister,, West Drayton, Middlesex UB7 7AH (GB); Vila Rifà, Enrique, 04009 Almeria, (ES)
(74) Representative: Hannke, Christian

(56) References cited:
- WO-A1-2006/057552
- US-A1- 2005 178 337
- DUC TUNG NGUYEN ET AL: "Development and reproduction of the predatory mite Amblyseius swirskii on artificial diets", BIOCONTROL, vol. 58, no. 3, 18 December 2012 (2012-12-18), pages 369-377, XP055266697, NL ISSN: 1386-6141, DOI: 10.1007/s10526-012-9502-y
- Ernesto Ragusa ET AL: "Effect of pollens and preys on various biological parameters of the generalist mite Cydnodromus californicus", Bulletin of Insectology, 1 January 2009 (2009-01-01), pages 153-158, XP055266692, Retrieved from the Internet: URL:http://www.bulletinofinsectology.org/p dfarticles/vol62-2009-153-158ragusa.pdf
- MARIA NOMIKOU ET AL: "Phytoseiid predators of whiteflies feed and reproduce on non-prey food sources", EXPERIMENTAL AND APPLIED ACAROLOGY., vol. 31, no. 1/2, 1 January 2003 (2003-01-01), pages 15-26, XP055266693, GB ISSN: 0168-8162, DOI: 10.1023/B:APPA.0000005142.31959.e8

## Description

The present invention relates to a method for rearing and/or increasing the activity of at least one population of a predator mite species of the family Phytoseiidae, wherein at least one rearing population of a predator mite species of the family Phytoseiidae and a first food source for this predator mite species are provided, wherein the rearing population of the predator mite species and the first food source are both located in a common housing, which has at least one opening which allows individuals of the predator mite species to leave the common housing.

Furthermore, a mite composition is disclosed comprising at least one rearing population of a predator mite species of the family Phytoseiidae and a first food source for this predator mite species, wherein the rearing population of the predator mite species and the first food source are both located in a common housing, which has at least one opening which allows individuals of the predator mite species to leave the common housing.

A further aspect of the invention is a method for protecting a crop by providing at least one rearing population of a predator mite species of the family Phytoseiidae and a first food source for this predator mite species in the crop, wherein the rearing population of the predator mite species and the first food source are both located in a common housing, which has at least one opening which allows individuals of the predator mite species to leave the common housing.

Members of the acarine family *Phytoseiidae* have provided the majority of the predators selected as biological control agents for use in protected crops. They are widely used in glasshouses and similar constructions, e.g. polythene structures. Currently, some seven species are being produced on a broad commercial scale worldwide. A summary of the use of these species is given by Griffiths (2003) and van Lenteren (2012).

The application of these agents to the crops is carried out using inundative releases. On small hectarages it will be done using hand held shaker bottles or blower machines. For larger areas and/or tall crops the blower will be mounted on a machine capable of holding a much larger reservoir of predators. For low herbage crops, a modified fertilizer spinning disc apparatus is used. The objective of these well-known techniques is to distribute the predators evenly throughout the crop at effective and economic intervals of time so that during the life of the crop the ratio of predator to pest will maintain a balance in favour of the predator.

A successful ratio is best, often only, achieved if a reasonable breeding population of the agent can be established in the crop before the first pests invade. However, if predators are introduced before the pest arrives, they will quickly die of starvation, especially if the crop itself is unable to provide nutrients, such as pollen or nectar. Many valuable crops, e.g. cucumbers, do neither have flowers with pollen, nor possess nectar sites, and so do not have an alternative nutrient source. Other crops, such as young sweet pepper plants or ornamentals, will have yet to flower. However, even if pollen or flowers are present, not all predators can use pollen or nectar as food sources.

Thus, a major drawback with current biological programs for protected crops is that the factors, which influence the arrival of the first pest populations, are complex, and to date neither the commercial producer, nor the grower, has been able to reasonably identify the timing of this event.

Usually, predators are applied to the crop using the "sachet technique", which was invented by Griffiths (Sampson et al. "The commercial development of an Amblyseius cucumeris controlled release method for the control of Frankliniella occidentalis in protected crops". The 1998 Brighton Conference - Pests & Diseases, 5B-4, 409-416). The sachet consists of a small paper envelope, into which is placed breeding populations of both the predator and its prey, together with a farinaceous food supply for the prey, plus a small quantity of commercial, bulking substrate composed of bran flakes or vermiculite particles. The envelope is sealed and provided with a small cardboard hook, which is used to hang the sachet on to a plant. A very small hole is pierced in the side of the sachet towards the top margin at the time of filling so that the predators can exit on to the leaf.

Since the prey, the astigmatid host, is the food for the predator, both predator and prey can breed whilst inside the sachet. The host usually belongs to a group of mites which are known as "food storage" species and are usually members of the Astigmata. As such they are conditioned to remain inside the sachet, since historically they are used to living in the dark, or in low light levels Thus, this host tends to stay inside the sachet along with its food supply and, in general, none or just a very few will emerge from the sachet, by accident.

This technique led to significant improvements. Since the host is a food source for the predators it is capable of living inside the sachet for some weeks. They will be well fed when they leave the sachet but if the pest population is not present when they emerge from the sachet, then in a short time they will starve, with very few, or no survivors if the pests remain absent. If this situation continues over a period of a few weeks the emerging predators will continue to die so that when the pests eventually arrive they do so into a virtually unprotected crop, where the pest will quickly become established, forming a stable breeding population in safety.

Many attempts have been made to solve the problem that on one hand if the pest is not present when the predators emerge on to a leaf, then these predators are extremely vulnerable to starvation. On the other hand, if the predators are not present when the pest arrives, the pests will grow fast and establish a stable population which will seriously disadvantage any necessary "fire-brigade" introduction when the pest eventually does arrive. Thus, when the sachets of the first introduction of the program are in place, and the pest is still absent, the ability to provide predators, as they emerge from these sachets, with an adequate food supply, has been identified as a possible solution to this problem. Accordingly, efforts have been made to provide additional food sources, which for the predators are alternatives to their natural foods (e.g. the pests) when they emerge on to an empty leaf. However, only a few materials are known or commercially available which could possibly solve this problem, at least, in part.

In the past, many attempts have been made to stabilize the population of predators by providing fresh or partially dried pollen grains, sterilized eggs of the flour moth *Ephestia kuehniella,* cysts (eggs) of the brine shrimp, *Artemia,* normally a food for ornamental, exotic fish species or artificial diets of various combinations as alternative food source for the predators. However, most of these systems could not be brought into practice. Most experiments to determine their usefulness have only been performed performed on a laboratory scale (e.g. petri-dish/leaf-disc experiments) or using a small numbers of potted plants, held in laboratory cages.

As an alternative food source *E. kuehniella* eggs give by far the best results. The eggs of this moth are known as food in large scale productions of certain species of the Trichogrammatidae (egg parasitoids) employed to control pests of field crops, such as the corn borer. However, because of concerns as to the effectiveness of control programs and cost of production, the use of these parasitoids is in debate. Further, where protected crops are concerned, high production costs leading to expensive end products is the major limitation in the use of biological control agents and the cost of *E*. *kuehuniella* eggs is the major factor seriously limiting their use as an alternative food. Moreover, the eggs of this moth are a good food for different insects, like the predatory bugs (*Hemiptera*), but not the preferred food for most of the predatory mites.

*Artemia* cysts are the encapsulated eggs of the brine shrimp *Artemia* spp., and when decapsulated form a basic food for exotic ornamental fish species, hence large quantities are sold across the world each year. However, the costs are relatively high. Thus, there is just one commercial product on the market, sold by one company which is recommended for use as a supplementary diet for use with the predatory bug (Hemiptera) *Macrolophus pygmaeus* in the absence of its pest prey, whiteflies. However, the company does not recommend this product as an alternative food for *phytoseiid* predators.

Because of the drawbacks associated with the alternative food sources mentioned above, the major effort in research and development of the use of alternative diets have been directed to the use of pollen. However, the results and conclusions of this research are contradictory. Some researchers conclude that, as an alternative pre-pest food pollen represents a viable proposition, whereas others defer. This is probably not due to different approaches in methodologies but by the fact that there are very different definitions and qualities of "pollen". "Pollen" actually represents an enormous number of specific entities and, in the context of an alternative food, should not be considered as a collective noun. The pollen profile for each flowering plant is unique, thus to one phytoseiid mite species a specific pollen may represent a natural food, whilst to another it will be factitious, unknown to it in its natural environment. Delise et al. (2015), when testing *Amblyseius swirski* on certain pollens rated: cattail 1^{st}; apple 2^{nd} and maize 3^{rd}, but when testing with *A. cucumeris* rated: cattail 2^{nd}, apple 1^{st} and maize 3^{rd}. In contrast to Oveja et al. (2012), who offered 21 different species of pollen as exclusive food to *A. swirskii.* Three species caused complete mortality. Three other species gave very poor development / reproductive results. These three species, plus the remaining species (18 species in total) proved to be similar to cattail pollen or an even better food source.

Such variation between preferences shown by predator species to different pollen species means, that the future selection of the right pollen as an alternative food will be difficult and time consuming.

Other drawbacks associated with pollen as an alternative food are in themselves major problems, for example:
- scarcity of a regular pollen supply for commercial usage, relative to the need to employ different pollen species,
- reliability of purchasing from an outside source,
- storage problems relating to fresh pollen, chiefly rapid protein denaturing,
- a sales provider of cattail pollen has estimated that if used as a viable diet for large predator populations it is expected to have a viable life of only about two weeks, before a new application is required,
- currently only one commercial pollen product is on the market. This product is cattail pollen (*Typha* sp.). Thus, only a very small market can be satisfied,
- pollen also provides a source of nutriment for many pest species, and
- in moist, warm environments, such as experienced by crops in certain countries, pollen will quickly become attacked by fungi.

The patent application US 2005/178 337 A1 discloses an amended release system for the prey mite *Tyrophagus putrescentiae* and the escape rates of predator and prey mites of this and older systems are compared.

From WO 2006/057 552 A1 a mite composition for rearing predatory mites of the species *Amblyseius Swirskii* on a host population of astigmatid mites is know. This composition is applied to the crop in sachets with an opening for the predators through which also few prey mites can escape.

Nguyen, D. T. et al.: "Development and reproduction of the predatory mite Amblyseius swirskii on artificial diets", BIOCONTROL, vol. 58, no. 3, 18 December 2012, pages 369-377, discloses that *Amblyseius Swirskii* develops better on *Carpoglyphus lactis* as a host than on cattail pollen under artificial conditions.

There is no guarantee that any predator introductory program known in the art which can develop and maintain a predator population over five weeks or even longer, even if introduced into the crop in suitably high numbers, if the crop is free of pests. Thus, the problem to be solved is to provide a cheap system which can be easily applied and by which a stable population of predators can be provided and maintained in the crop for such a long time period in the absence of any pests.

This problem is solved by the subject matter of the independent claim 1.

Accordingly, a major aspect of the invention is a method for rearing and/or increasing the activity of at least one population of a predator mite species of the family Phytoseiidae comprising the steps:
- providing at least one rearing/breeding population of a predator mite species of the family Phytoseiidae and an initial food source for this predator mite species, wherein the rearing population of the predator mite species and the first food source are both located in a common housing, which has at least one opening which allows individuals of the predator mite species to leave the common housing, wherein optionally the common housing is designed as a sachet of determined size, as known from the prior art (Sampson, C. "The commercial development of an Amblyseius cucumeris controlled release method for the control of Frankliniella occidentalis in protected crops. The 1998 Brighton Conference - Pests & Diseases, 5B-4, 409-416),
- providing a second food source for the predator mite species, wherein the second food source comprises at least one population of a mite species belonging to the Astigmata,
- arranging the common housing on a plant,
- placing the second food source on the same or a different plant, but external of the common housing and preferably spaced apart relative to the pattern of distribution of the common housing.

As known from the prior art namely, the "sachet technique", the common housing can be in the form of a sachet. Within such a common housing, individuals of a predator mite species of the family Phytoseiidae, can be provided together with a "first" food source. The initial food source (e.g. the host) can be a mite species belonging to the Astigmata. Suitable combinations of hosts and predators are known from the prior art and can be selected by a skilled person. The most suitable is usually a combination depending on the target crop, the expected target pest or pests, climatic conditions, local regulations and other factors. The common housing has at least one opening, through which individuals from the predator mite species can emerge. The host, which is preferably selected from a "stored food mite species" will usually remain in the dark inside the common housing. If a sachet is used, the host will remain in the dark inside with its farinaceous food and the preferred carrier. Within the carrier, the host mites can hide and/or lay eggs.

The common housing (preferably the sachet) is arranged on a plant If a sachet is used it is hung by its small cardboard hook on a plant. Thus, the predators can emerge and protect this plant and preferably extend their territory to neighbouring plants. Adult pests or a specific developmental stage in the life-history of this pest can be destroyed by the predator mites. Thus, the pest cannot develop further and is held at a low, inadequate, population number. The aim is to reduce the development of the pest population to below the economic damage level which will be a low population number.

The method of the present invention differs from the known systems in that in addition to the initial food supply of astigmatid mites for the predators, provided inside the common housing, a second food source for the predator mite species is also provided, preferably on the plant, exterior to the common housing. This second food source comprises at least one (preferably breeding population) of a mite species belonging to the Astigmata. This species also acts as a (second) food source for the predator and may represent a different species to the host (the first food source) inside the common housing (e.g. the sachet), but in a preferred embodiment it is the same species as in the common housing (or sachet) and even more preferred also the same species on which the predators were reared commercially. This is usually a further advantage of the system since the predator's diet remains unchanged from that of its mother for all of its life. In this case, the system is particularly effective since the predators will never experience a change in diet.

A further difference of the present invention with respect to the known methods is that this second food source is placed in or on the same or a different plant but external to the common housing and preferably spaced apart with respect to the positioning of the common housing(s) in the crop. Thus, this second food source can be an supplementary instant food source for those predator mites which have emerged from the common housing, Thus, even when there is no pest present, the emerged predators will find an acceptable food source, enabling it to develop further and preferably allowing a stable population of the predators which can be maintained within the crop.

Predator emergence from the sachets (or other common housings) usually follows a particular pattern. In the presence of a good supply of prey individuals inside the sachets, predator emergence is related to the number of gravid females inside the sachet (carrying one or two eggs, waiting to be laid). In this condition they instinctively exit through the small hole looking for an uncrowded site, which naturally will be outside the exit hole, so few individuals are released, and emergence is slow. Sachets of this type are called "Controlled Release" sachets. From a "healthy" sachet of this type, only small amounts of female predators will emerge slowly as they become gravid, with one or two eggs in their body. This trickle emergence can be maintained over a three to six week period, hence the term "Controlled Release". Preferably the second food source is provided early during this time and preferably replenished if and when needed, depending on the course of the pest invasion. Preferably the second food source is provided to the crop relative to the distribution pattern designed for the sachets. Preferably the second food source is placed on the leaves of the crop at the same time as the sachets are hung on the plants, or 1 day to 2 weeks, preferably 2 to 7 days after the sachet is positioned in the crop. Preferably in the absence of any pests, the initial introduction of the second food supply will occur 2 - 14 days after the common housing is positioned in the crop.

However, as the prey population inside the common housing begins to get low, then the subsequent shortage of food will encourage many females to emerge on to the plant. Preferably the second food source is provided prior to this event, or, at the latest, one or few days after this event has occurred. Subsequent introductions of the second food source will, of course, depend on various factors, especially the food shortage. It is necessary to introduce a regular program of cheap second food source at identified intervals depending on these factors. This preferable to a complicated scouting program and will usually cost less.

In a preferred embodiment, introductions of the second food source will, of course, depend on various factors, especially those related to predator food shortages or changes in the physical environment which can occur within glasshouses. Thus, it is necessary to introduce a regular program of the second food source to respond to such situations.

In a further embodiment, when the common housing is being prepared the ratio of predator to prey can be altered to suit these conditions which may occur in the crop. The skilled person will know that depending on the composition of this ratio, the predators will have different patterns of emergence. Thus if a sachet introduction is required to deal with an active prey population, or there are signs of an imminent pest invasion, the ratio can be altered from one for a "Controlled Release" system to give a mass exodus of predators over a short period of time. This is termed a "Quick Release" system.

Thus, preferred embodiments of the present invention are those where the common housing comprises either a "Controlled Release" system or a "Quick Release" system, depending on the event for which it is intended. Thus, a large, thriving healthy population of predatory mites is guaranteed to be present in the crop, preferably at the top of the plants at all times, and with or without the presence of the pest. Thus, the present invention gives the grower the best possible chance of achieving a non-pesticide control.

In a preferred embodiment, the second food source is placed on a leaf of a plant. Even more preferred is that the second food source is placed on the top of a leaf. Of course, the second food source can alternatively or additionally also be applied on the ground.
To attract the predators to those parts of the plant / crop which are prone to be infested by a pest, the second food source is preferably placed on a leaf which is prone to be infested by a pest or which is on a plant which is liable to be infested by a pest, and is but a short distance away. How short this distance should be depends on the plant/crop and the activity of the predator mites. Preferably the second food source is placed in a distance of less than 50 cm, preferably less than 30 cm, most preferably less than 15 cm to a part of the plant which is prone to be infested by a pest.

In a preferred embodiment of the present invention, the common housing (e.g. the sachet) and the second food source are located in the same crop. More preferably the common housing and the second food source are located on the same or neighboring plant(s). A suitable distance between the common housing and the second food source should be selected depending on the plant/crop and the activity of the predator mites. For some very active predators with a wide range of activity, it can sometimes be beneficial to place the common housing external to the crop. This may make it easier to obtain a better distribution pattern relative to the positioning of the pest population.

In a further preferred embodiment of the invention, the second food source comprises a third food source. If the second food source comprises a rearing astigmatid population and no sufficient food source for these mites is present in the crop, a third food source is advantageous to maintain a high number of astigmatid mites in the crop as (second) food source for the predators. In a further preferred embodiment the third food source comprises wheat germ and/or farinaceous material. Even more preferably the third food source comprises the food and/or carrier system on which the Astigmata are reared. Thus, when the second food source is prepared for distribution on the leaves of the crop it will be mixed by the technicians to contain a sufficient supply of food judged to keep the astigmatid population alive and, in part breeding, whilst on the leaf. Of course, the third food source can be applied more often than the second food source. Such a further application of the third food source is preferred if the current supply becomes depleted, leaving the second food source (the astigmata) in danger of starvation.

Since commercial crop patterns, especially 'rowed' crops, as well as plant configurations can limit the spread of the predator which is essential for good control, in a preferred embodiment of the present invention a plurality of common housings, and a plurality of second food sources are located and maintained in the crop. The density of common housing present in the crop is preferably between 1000 common housing /m² and 1 common housing/25 m². More preferably, the density is between 2 common housing/m² and 1 common housing/5 m², most preferably between 1 common housing/m² and 1 common housing/2 m². However, even higher densities of common housings in the crop are possible, especially when the material is sprayed onto the crop.

Independent of the density of the common housing in the crop, the density of the second food sources in the crop is preferably between 1000 second food sources /m² and 1 second food source/25 m². More preferably, the density of the second food sources is between 3 second food sources /m² and 1 second food source/5 m², most preferably between 1 second food source/m² and 1 second food sources/2 m². However, even higher densities of the second food sources in the crop are possible.

In a preferred embodiment, the second food source is sprayed onto the crop. In this embodiments, a vast number of small spots of second food sources are applied to the crop. Depending on the method of application these spots of second food supply can be located on leaves and other parts of a plant such as stipes, stalks, flowers etc.. These spots can also be located on the ground.

To provide a suitable density of predators and to establish a stable population of predators it is preferred that each common housing comprises between 1 and 5000 individuals of the predatory mites. Preferably between 50 and 1000 individuals, more preferably between 100 and 500 individuals, most preferably about 250 individuals of the predatory mites are located in each common housing. The number of individuals can be related to the required density of the common housing in the crop, the activity of the predatory mites, the species (of predator) and their reproductive rate.

In a further preferred embodiment, the density of the individuals of the mite species belonging to the Astigmata in the second food source is between 500.000 and 50 million individuals/litre. Using a density in this range permits the application of a suitable number of individuals per spot on the plant. In a further preferred embodiment, the density of the individuals of the mite species belonging to the Astigmata is between 10 and 30 million individuals/litre, most preferably between 15 million and 20 million individuals/litre. At these densities, it is easy to apply a suitable number of individuals to each spot on the plant using known techniques. Independent of the density, in a preferred embodiment of the invention, a volume between 0,01 and 50 ml of the second food source is placed on one spot. Preferably between 1 and 20, most preferably between 5 and 10 ml of the second food source is placed on the same spot. These volumes can be located and applied on the plant by known techniques. They include known apparatus such as automatic or manually operated sprayers, syringes, dropping devices or spoons.

To avoid any negative effects caused by the second food source to individual plants or the crop, it is preferred that the mite species belonging to the Astigmata are selected from those species currently known to be harmless to agricultural crops.

In a preferred embodiment of the invention, at least one of the mite species belonging to the Astigmata is selected from a group of mite species comprising *Carpoglyphus lactis, Tyrolichus casei*. *Thyreaphagus entomophagus* and *Lepidoglyphus destructor.* These species have been identified as acceptable food source for specific predator mites. Furthermore, these species are known to cause no harm to a wide range of commercially grown plants or crops.

In a preferred method of the present invention, at least one of the mite species belonging to the Phytoseiidae is selected from a group of mite species comprising *Amblyseius swirskii, Typhlodromus montdorensis, Neoseiulus californicus, Amblyselus andersoni, Neoseiulus cucumeris* and *Typhlodromalus* (=*Amblyseius*) *limonicus.* These species have been identified as efficient predators which can be used against a plurality of pests. Furthermore, these species have never been identified as causing harm to a crop plant.

Furthermore, a mite composition comprising at least one rearing population of a predator mite species of the family Phytoseiidae and a first food source for this predator mite species is disclosed, wherein the rearing population of the predator mite species and the first food source are both located in a common housing, which has at least one opening which allows individuals of the predator mite species to leave the common housing, wherein the mite composition comprises a second food source for the predator mite species, wherein the second food source comprises at least one mite of a mite species belonging to the Astigmata, wherein the second food source is located outside the common housing and preferably spaced apart with respect to the common housing. Preferably the second food source is at least one population of a mite species belonging to the Astigmata. More preferably the population of the mite species belonging to the Astigmata is a stable, breeding and/or growing population. As described above with respect to the method, such a system can provide a further food source for the predators situated exterior to the common housing. Thus, the population of the predators can be a stable and/or even growing population. Thus, large numbers of predators will be available to attack when a pest invades. The aim is for predators to prevent the pest from establishing a population in a crop.

The mite composition comprises at least one population of mite species belonging to the *Astigmata* in order to provide an in-crop food source for mite predators of the *Phytoseiidae* as well as a method for the application of at least one population of mite species belonging to the *Astigmata* in order to provide an in-crop food source for mite predators of the *Phytoseiidae,* when being used as biological control agents to resolves long-term problems associated with the history of employing biological control agents on crops grown in glasshouses and polythene structures. It also opens the possibility to further develop biocontrol programs in open field crops such as orchards.

In a preferred embodiment of the system, the second food source comprises a third food source, wherein the third food source is a food for the mite species belonging to the Astigmata. This third food source can feed the mites in the second food source. Thus, the population of the mites of the second food source stays alive for a long time period. Preferably this period is long enough that it includes the time up to the time of the pest Invasion. Preferably, a new or additional third food source is applied again when or before the previously presented third food source is depleted.

In a preferred embodiment of the system, the common housing is a sachet. More preferably this sachet comprises a hanger for hanging the sachet on or in a plant. Such a hanger can be a hook which is connected with the sachet e.g. by a filament or fiber. Also brackets, clips, clamps, adhesives or others systems can be used for attaching the common housing to the plant. In bigger plants (e.g. trees or bushes) also invasive methods like nails or screws can be used for attaching the common housing to the plant.

In a preferred embodiment of the system, the predator mite species is selected from a group comprising *Typhlodromus montdorensis, Amblyseius swirskii, Neoseiulus cucumeris, Neoseiulus californicus, Amblyseius andersoni* and *Typhlodromalus* (=*Amblyseius*) *limonicus.* The mite species belonging to the Astigmata is selected from a group comprising *Tyrolichus casei, Carpoglyphus lactis, Thyreophagus entomophagus* and *Lepidoglyphus destructor.* An even more preferred embodiment of the system is that wherein the combination of a predator mite species with the mite species belonging to the Astigmata is selected from a combination of *T. entomophagus* with *T. montdorensis,* or *C. lactis* with *A. swirskii.* These combinations have been shown to provide a stable system which is not harmful to a wide range of plants or crops, and is effective against a plurality of pests, and is able to establish a stable system over a long time period of time, last from two weeks to two month or even longer.

Further advantages, aims and characteristics of the present invention are explained with reference to the appended drawings and the following description in which embodiments of the method and/or the system are illustrated and described by way of example. The figures show:
- Fig. 1:: The mean number (± SE) of *A. swirskii* individuals per leaf in the different treatments and sampling dates, over ten weeks, in a cucumber crop transplanted in October 2013,
- Fig. 2:: The mean number (± SE) of *T. montdorensis* individuals per leaf in the different treatments and sampling dates in a cucumber crop transplanted in October 2013 (* = means significantly different with p= 0.005), and
- Fig. 3:: A schematic illustration of an embodiment of the present invention.

One example for a method of application by timed, inundative releases, in which the substrate, made up of a mixed population of predator/prey, is sprinkled on various leaves at different levels in the crop, preferably onto individual leaves, from a 0,250 to 50 litre volume cardboard or paper container. It is carried out by hand or by various types of hand held "blower" machines, or by a modified fertilizer spinning disc apparatus. Also "sachets" can be used. Another major application method is where the common housing is in the form of a sachet, in which the predatory mite species of the family Phytoseiidae and the first food source are located in this common housing, which has at least one opening which allows individuals of the predatory mite species to leave the common housing.

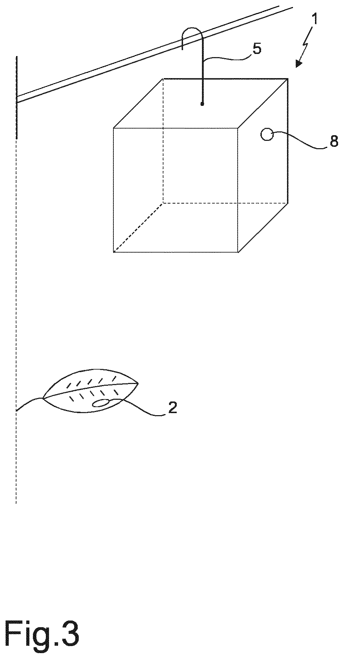

An example of a system comprising a common housing (1) with at least one rearing population of a predator mite species of the family Phytoseiidae and a first food source for this predator mite species both located inside the common housing (1) is shown in Fig. 3. In this common housing (1) the predators as well as the prey mites are located (all mites not shown). Furthermore a food source for the prey mites is provided (not shown). The common housing (1) has at least one opening (8) which allows individuals of the predator mite species which are located in this compartment (4) to leave the common housing (1). If the first food source is also a mite species (e.g. an astigmatid mite species) this first food source preferably located in areas, where conditions are provided which allows the mites to establish a stable population. These areas can contain bran and/or wheat germ and/or other products which provide a food source and/or cavities for hiding and/or egg-laying. Furthermore a second food source (2) for the predator mite species is shown. This second food source is located outside the common housing (1) and preferably spaced apart with respect to the common housing (1). As shown, this food source (2) is preferably located on a leaf. Preferably this second food source (2) comprises a rearing population of a mite species belonging to the Astigmata. Even more preferably the mite species of the second food source (2) is identical to the mite species which is used as the first food source inside the common housing (1).

The second food source for the predator mite species is provided in the same crop where the predatory mites had been previously introduced, wherein the second food source comprises at least one rearing population of a mite species belonging to the Astigmata. The second food source is provided after the introduction of the predatory mites. The second food source is sprinkled on various leaves at different levels of the crop onto individual leaves, from a 0.250 to 50 litres volume cardboard or paper container. Delivery is by hand or by various types of hand held "blower" machines, or by modified fertilizer spinning disc apparatus.

Usually, a plurality of common housings (comprising mixtures of mite predators and their astigmatid factitious preys) and a plurality of second food sources are located in the crop. The density of common housings depends whether the material is introduced using loose material or sachets, using machinery (blowing machines and others) or by hand. When using sachets, each sachet will be a common housing, when using loose material, each spot of the mixture of predatory accompanied by their first food source will be a common housing. When using machinery, the density of both common housings and second food source in the crop will be much higher than when using hand held methods. Preferably the sachets are used to introduce the predatory mites onto vegetable crops while loose material, preferably using machinery is sprinkled onto ornamental plants In general, the density of common housings in the crop is between 1000 common housing/m² and 1 common housing/25 m². Preferred embodiments are defined above. The density of second food sources in the crop (releasing points) is preferably 1000 second food sources /m² and 1 second food sources /25 m². Preferred embodiments are defined above.
One of the main advantages of the introduction of this in-crop food source is that it can be placed on the leaves which are liable to be infested by the pests. Thus, the predators can be lured to concentrate in these areas. The in-crop food source can increase the populations of predatory mites as a consequence of a numerical and functional response of the predatory populations. The increase of the populations in the desired parts of the plant that are more susceptible to be infested by the pest and/or where the pest can make the most important damages will prevent crop damage and so improve the success level of the biocontrol program. For instance, for many crops the young parts of the plants are more prone to be infested by thrip. Thus, the introduction of the in-crop food source onto the young leaves can encourage the movement of the predators to these parts of the plants, favoring predator development, thereby increasing the predatory populations in these areas and, as a consequence decreasing the risk of damage produced by said pests.

Another further advantage of the invention is the provision of an in-crop food source that can remain available for the predatory mites on the leaves of the crop for longer time than the other current alternative food sources applied to feed predatory mites, e.g. pollen (commercial brand "Nutrimite"). Depending on the climatic conditions, the astigmatid mites can survive on the leaves for about three weeks in winter conditions in the Mediterranean plastic greenhouses. The astigmatid species selected as a food source are known to have no record of having caused commercial damage to the crop on which they will be distributed. Thus, they will survive for longer time if some food is supplied whilst on the leaves.

For this reason it is preferred that a third food source, wherein the third food source is a food for the mite species belonging to the Astigmata, is applied onto the crop. Most commonly, mass rearing's of the astigmatid species is produced using foods of farinaceous nature, yeast, dried fruits and sugars among others. Preferably the productions use bran as a substrate. The third in-crop food source preferably comprises at least one of the food sources that is used in the mass rearing of the astigmatid species. In the current invention, most preferably bran is used as the substrate as a bulking agent to help introduce the selected astigmatid mite populations. Bran is not a high source of nutrients, so other food materials may be added to the mixtures as a third-food source. A portion of flaked wheat germ, which is by far the best diet for any stored product species, may be introduced, preferably in the form of very fine particles which enables it to stick to leaves which are more or less lying horizontal. It can also be found inside the curled bran flakes. This system further allows that, prior to use, the diet can be adjusted, depending on the number of days it is intended to use the food source material, ad lib or in controlled amounts.

Experiments have shown that these conditions give a very suitable supply of nutritious food for the astigmatid mites (the second food source).

Thus the advantages of the present invention can be summarized to be (not all features have to be present for all embodiments):
1. The danger of an insecure supply when purchasing the alternative food, such as brine cysts, and especially pollen, is obviated since the active ingredient (the second food source, namely live or dead astigmatid individuals) can be produced in-house under controlled conditions.
2. The second food source is obtained as a bi-product of the commercial production of phytoseiid predators, being the discrete high density populations of the factitious host which are grown in large quantities to rear the predators.
3. Since every commercial production system will always produce a surplus of astigmatid prey because seasonal demands vary very quickly. The surplus of prey is always on hand. Previously it was often destroyed, since commercial productions must always run above estimated requirements to deal with unexpected surges in pest invasions. Now this surplus is a useable product.
4. Since the commercial production systems are more or less a continuous operation, the second food source can be produced very cheaply compared to buying in suggested alternative products (e.g. pollen, cysts).
5. The predator for application on the crop can be matched to the astigmatid species to which it is most suited. Thus, when the "third food" system is in operation, the phytoseiid species will arrive on the green leaf where it will prey on and eat the same species of factitious host it has enjoyed as its diet since the beginning of its life.
6. It has been shown that for both astigmatid prey and pollen grain food, the phytoseiid can have specific food choices, whichin the case of pollen has been shown may cause problems when the predator is released on to the crop. When a specific astigmatid is the host for the predator from the first day of its life (to long after its release into the crop), this no longer becomes a problem.
7. Experimental data shown below indicate that application of the astigmatid species need only to be applied at intervals of four weeks if pests have yet to invade the crop. For pollen, the sole commercial supplier of cattail (*Typha sp.*) pollen states that in the same situation pollen has to be applied in two weekly intervals.
8. Unlike pollen, astigmatid species, at any stage of their development, which are currently used in commercial biological programs, are not attractive to thrip.
9. Experimental data shown below indicate that the presence of the factitious host does not preclude the phytoseiid mites from predating thrips. When pollen is the alternative food supply, especially where the predator is known to be a pollen feeder (e.g. species of *Euseius*), then to some extent thrips are less attractive as prey for certain species of phytoseiid mites.
10. In the event that the crop pest is an astigmatid species, for example the species *Tyrophagus similis,* which is a pest of protected spinach crops, then the applicant can introduce dead individuals of this species as a second food source, whereas live populations of this species can be used as prey to rear the predator and as first food source. Thus, one continuous diet for the predator is provided.

### Experimental data

### Experiment 1

First, experiments were made to determine whether the soil beneath certain glasshouse grown crops would offer a better environment to a population of a suitable *astigmatid* species when compared to a bare leaf. A first question to be solved was whether they would survive and possibly increase. Therefore, a simple experiment was set up to determine if any such species deemed suitable could survive in the soil substrate making up a chrysanthemum bed. Ground material, from such a bed, was collected and placed in 5 l plastic boxes. Mites, without predators, were sprinkled on the surface of the soil. *Astigmatid* populations were mixed with bran and packaged on 1 l bottles with concentrations of about 30 - 40 millions / litre, and a volume of 10 ml of this prey population was spread at the top of the ground material, and the boxes sealed with a mesh to offer ventilation. Two boxes were set up per each tested species. In one of the boxes 5 ml of bran and 20 ml of vegetable compost were previously spread over the top of the ground as additional food to support the development of the prey mites. All boxes were placed in a climatic chamber at 25°C and 70% RH (relative humidity). Sampling was made every 2 - 3 days over a total of 3 weeks. Each time, about 40 ml per box were collected and observed under a binocular microscope.
The results of these experiments are shown in Table 1. It can be seen that only a few individuals of one species survived. This was *Tyrophagus putrescentiae,* which is a plant dweller whilst the other five are not, being confined to food storage environments. Further, it has already been pointed out that it can cause plant damage. The other five species tested died after a few days even when flakes of bran and/or vegetable compost were applied to the top layer of the soil as a food to help them develop.

**Table 1. Observations of the development of different species of prey populations at the top of Chrysanthemums' ground material during 3 weeks.**

| Species | Week | | |
|---|---|---|---|
| | 1 | 2 | 3 |
| *Tyrophagus putrescentiae* | ++ | + | - |
| *Thyreophagus entomophagus* | - | - | - |
| *Carpoglyphus lactis* | - | - | - |
| *Acarus siro* | - | - | - |
| *Tyroglyphus casei* | - | - | - |
| *Glycyphagus destructor* | - | - | - |

| | | | |
|---|---|---|---|
| ++ = individuals of different stages and eggs present, + = only few individuals observed but no eggs present, - = less than 4 individuals alive | | | |

Additionally, an experiment was set up to evaluate if the release of *T. putrescentiae* at the base of a chrysanthemum crop to see if it could support the development of *T. montdorensis* and thus the control of thrip populations in these conditions.

The results showed that this methodology did not improve the development of the predatory populations and did not favor the control of the thrip. There was no significant difference in the abundance of predatory and pest populations when the prey mites were applied or were not applied to the ground before the introduction of the predators.

### Experiment 2

It was decided that the soil option alone was not tenable and, since the history of the use of pollen grains or *Artemia* cysts did not offer a viable solution, a detailed "top leaf trial" was implemented. A short resume of this trial is shown below. The complete scientific account of the trial is provided later.

Two pairs of "predator plus prey" were selected: *Typhlodromalus montdorensis* and the factitious prey *Thyreophagus entomophagus,* and secondly *Amblyseius swirskii* with the factitious prey *Carpoglyphus lactis.* Both predators are suitable to target the thrip pest *Frankiniella occidentalis* and the whitefly *Bemisia tabacci*.

Major results were:
- When prey mites were used as a second food on the top leaves, the *A. swirskii* population was five times larger than in the control.
- On the last day of counting, 25 days after the last release of prey mites, thousands of *A. swirskii* eggs were found laid in the vicinity of some residual clumps of prey mites.
- More than 500 predators were counted on some leaves where prey mites had been introduced.
- For *T. montdorensis,* similar egg clusters and up to 500 predators per leaf were present where live prey still existed.
- Where prey mites had been introduced, the absence of thrip larvae, which are the principal predated stage, shows the success of this technique.
- When high infestations of the thrip pest came in from outside the greenhouse *A. swirskii* significantly reduced these pest populations compared to the numbers found in the control plots.
- The number of predators on the control plants was about that which one would expect on a cucumber crop in a southern Spanish winter. These predatory populations were not able to control the high infestation of thrips, which commonly occurs in January.

### Experiment 3

This experiment was made to evaluate the seasonal abundance of predatory mite species and the control of the main pests, thrips and whiteflies, with or without *astigmatid* prey populations added to the top of plants (grown in rows) as a supplemental source of food.

This experiment was conducted on a cucumber crop in an experimental greenhouse situated in the grounds of the Research Station Fundación Cajamar Las Palmerillas, which is based in Almeria, where the main bulk of greenhouses for vegetal production of Europe is concentrated. The experimental plastic greenhouse of 630 m² is made with the typical style of "parral" that is used in most of the protected crops of the south of Spain and other Mediterranean areas, which do not have heating or humidifier systems. A cucumber crop of variety "Valle" was planted on October 21^{th}, 2013. Density of plants was 1.0 plants/m². The plantings resembled that for a commercial crop.

Two predatory mite species were evaluated, *Amblyseius swirskii* and *Typhlodromips montdorensis.* A total of six treatments were established: *A. swirskii* + prey mite populations at the top of plants as complementary food, A. *swirskii* + pollen, *A. swirskii* without additional food added (control), *T. montdorensis* + prey mites at the top of plants, *T. montdorensis* + pollen and *T. montdorensis* without additional food added (control). Each treatment was established in separated rows, using a randomized block design with six repetitions (rows).

Both predators were introduced using a quick release sachet system. The sachets initially contained about 250 individuals of predatory mites/sachet which were hung on the plants at a ratio of 1 sachet every two plants, giving a release ratio of about 125 individuals / m². The sachets were introduced on November 27^{th} (week 47 of the year). Pollen or prey mites were introduced, according to the treatment, the following week, and then twice more at 15 day intervals.

Two species of prey mites were used: *Thyreophagus entomophagus* to feed *T. montdorensis* and C. *lactis* to feed *A. swirskii.* Both mites were packaged in bottles of 5 using bran as a carrier material, with a density of about 15 - 20 million individuals/litre. To spread the material at the top of the leaves a small spoon was used. A total of 8 ml (1 spoonful) was placed at the top of one leaf per two plants. Fresh multifloral pollen collected by honeybees was stored frozen and later defrosted the day before using. The pollen was mixed with water, shacked vigorously and then the mixture was sprayed at the top of a layer of vermiculite, so that all the pieces of vermiculite were covered by individual pieces of pollen. The vermiculite plus pollen was packaged in 5 litre bottles. A quantity of 8 ml was distributed at the top of the plants following the same methodology as described for the prey mites.

Sampling of the populations of predators and pests was completed every week. The total number of individuals (adults and nymphs) of predatory mites and thrips, as well as the number of larvae, pupae and adults of whiteflies were counted on three leaves (at basal, medium and high levels) per plant from eight randomly selected plants per treatment. The numbers of thrips and *phytoseiid* predatory mites were analyzed using an ANOVA per each sampling date and means were separated using a Duncan test (SAS System 9.0).

### Results

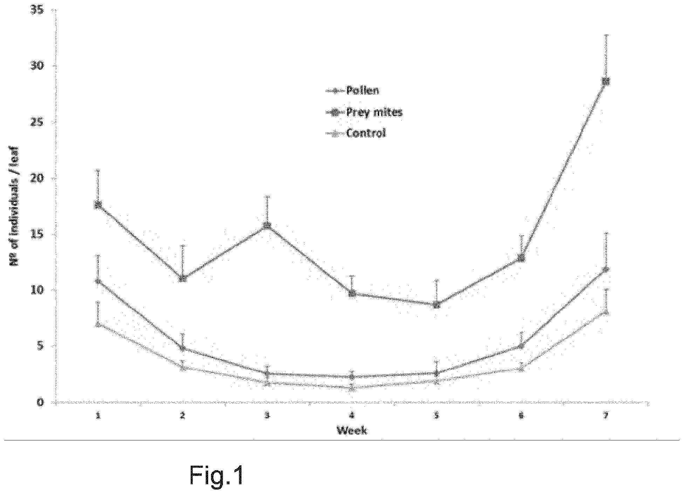

The densities of populations of *A. swirskii* in the three different treatments are shown in Figure 1. There were significant differences among treatments (F = 24.85; p < 0.0001) and among sampling dates (F= 15; p < 0.0001). There were higher populations of predatory mites when prey mites were added to the top of the leaves compared to the other treatments, with 4 to 5 times more populations compared to the control. Figure 1 shows the mean number (± SE) of *A. swirskii* individuals per leaf in the different treatments and sampling dates, over ten weeks, in a cucumber crop transplanted in October 2013.

Twenty-five days after the last introduction of prey mites had been made to the top leaves, live populations of this prey could still be observed on the underside of the leaf. This showed that the food remained available for a considerable time, so that the introduction of prey mites could on a commercial scale probably be recommended to be done every three weeks instead of every two weeks, at least in southern Spanish winter conditions.

Interestingly, on the last sampling date, 25 days after the last introduction of the prey mites, thousands of eggs of *A. swirskii* could be counted surrounding the small groups of residual prey mite populations. On some of these leaves, where the prey mites remained alive, the amount of nymphs and adults of *A. swirskii* were so high that it was not possible to count them properly (> 500 individuals per leaf).

Higher populations of *A. swirskii* were counted compared to the control scenario when pollen was added, but never in all the counts, and only on some dates. Large amounts of eggs of *A. swirskii* were never observed, even under the leaves where patches of pollen had been introduced.

The number of individuals in the control treatment were lower than the treatment where prey mites were added, with densities that are very typically found on southern Spanish cucumber crop in the winter period, when growers are following an integrated pest management, including use of compatible products to control the diseases present during this period of the year.

The populations of thrip and whiteflies, which are the main pests of cucumber, were at low levels during most of the trial period, with no damages to the fruits. During the first 5 weeks, the populations of thrips were between 0.5 and 1.5 individuals per leaf, with no differences between treatments, but a high infestation of thrip developed during the last two weeks of sampling.

An increase of this pest in January is a common situation in the cucumber crops of the southern Spain and can cause heavy damage to the fruits, at a time when predatory populations are at low levels. Then, very often chemical applications are made that decreases the *phytoseid* populations even more, disrupting the biological control.

In this trial, populations of thrip remained at significantly lower level on plants where prey mites had been added as a complementary food, compared to the control plot (Table 2).

Particularly remarkable was the fact that almost no thrip nymphs (the preferred target of the predator) were counted in the treatment with prey mites added, only adults, showing a very good control of the pest despite high infestation arriving from outside of the greenhouse. By contrast, nymphs and adults were present on the control plot in significantly higher populations of thrips. Populations were intermediate on the plants where pollen was added, but without significant differences compared to the control. Mean (± SE) number of larvae and adults of trips per leaf counted on January 21^{th}, 2014, on a cucumber crop under three different treatments are shown in table 2. Results are shown for plants where prey mites had been released, plants where pollen had been released and the control plants with no alternative food for *phytoseid* mites supplied (means followed by same letter were not different with p = 0.005).

**Table 2**

| | | |
|---|---|---|
| Prey mites | 3.7 ± 0,60 | a |
| Pollen | 5.1 ± 0.51 | b |
| Control | 6.0 ± 0.72 | bc |

Table 2: Mean (± SE) number of larvae and adults of trips per leaf counted on January 21^{th}, 2014, on a cucumber crop under three different treatments: plants where prey mites had been released, plants where pollen had been released and the control plants with no alternative food for *phytoseiid* mites supplied (means followed by same letter were not different with p = 0.005).

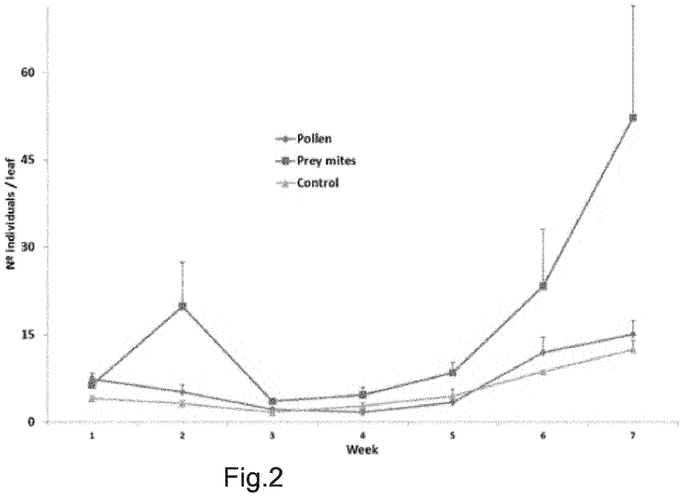

The densities of populations of *T. montdorensis* in the three different treatments are shown in Figure 2. There were significant differences among treatments (F = 10.09; p <0.0001) and sampling dates (F = 8.99; p< 0.0001), and there was interaction between both variables (F = 2.57; p = 0.004), probably explained by different results obtained with pollen on different dates. Figure 2 shows the mean number (± SE) of *T*. *montdorensis* individuals per leaf in the different treatments and sampling dates in a cucumber crop transplanted in October 2013 (* = means significantly different with p= 0.005). On four counting dates there were 2 to 5 times higher populations of predatory mites when prey mites were added to the top of the leaves compared to the other treatments. The prey populations (*T. caseientomophagus)* were alive on the underside of the leaves up to four weeks after their introduction, thus remaining as an alternative food for the predatory mites for a long time. The last week of sampling, hundreds of eggs of *T. montdorensis* and more than 500 individuals (nymphs and adults) were counted on the leaves where prey populations had been introduced about 25 days before and where the prey populations were still alive.

The populations of *T*. *montdorensis* developed higher populations than *A. swirskii* the last two weeks of sampling, coinciding with the infestation of trips, and they offered a better control of this pest. The numbers of thrips per leaf were similar among treatments with *T. montdorensis,* and remained at lower levels compared to the plots with A*. swirskii,* with mean numbers between 0.2 and 2.2 individuals / leaf during all the trial, and means between 1.4 and 2.2 individuals / leaf the last 2 weeks.

### Experiment 4

The aim of this study was to evaluate the increase or otherwise of the abundance of predatory mite species distributed on Chrysanthemum crops when an additional release of astigmatid prey mites as a second, supplementary food source is applied to the top of the plants.

This experiment was conducted in a commercial greenhouse crop of 3 hectares, situated in the southeast area of Holland. Traditionally, commercial crops of Chrysanthemums are transplanted as small plants in beds of approximately 1000 m². For the trial, two patches of 1000 m² were selected and the plants were transplanted on December 11, 2015. Density of plants was about 60 plants per m², which is the standard commercial procedure.

Two treatments were evaluated: releases of *Typhlodromips montdorensis* (control) and releases of *T*. *montdorensis* + an additional release of the prey mite *Thyreophagus entomophagus* as the secondary food source. Each treatment was applied to one of the two plots of 1000 m².

The released predatory mites were mass reared in the production facilities of Agrobio. This is a commercial product (MonControl) which contains the Phytoseiidae predator *Typhlodromalus. montdorensis* with the prey mite *Tyroglyphus casei* as its primary food source, together with a mixture of bran and vermiculite as a carrier material. The second food source released into one of the trial plots, is *T. entomophagus,* which is also reared in the facilities of Agrobio. This population of the prey mite was supplied with yeast and wheat germ as food sources, plus bran as a carrier material.

Both the trial units were introduced onto the top of the crop using a machine that has been develop specifically to spread loose material containing live populations of mites. The machine consists of a hopper, fitted with a system to control the quantity of material allowed to fall through a hole at the bottom of the hopper on to a modified fertilizer spinning-disc apparatus. Trials were carried out aimed to guarantee a distribution spread of material to give a 1.6 m circle on to the top of the plants, whilst maintaining the minimum possible mite mortality.

Each treatment used a total of 10 l (2 bottles of MontControl, each with 5 l) of the predatory mites *T. montdorensis,* introduced into the crop every week, always on Tuesday. The density of predatory mites was 25.000 individuals (nymphs and adults) per littre. This means that a ratio of 250 individuals per m² were released every week. The material was spread evenly so that about 5 small clumps (spots) of the substrate containing the predatory mites fell on to the top of the leaves of any plant. The second trial unit, the in-crop food source containing the populations of the prey mite *T. entomophagus,* was also released once every week, but always on a Thursday. The introduction rate was 10 l of material with a density of about 15 - 20 millions of nymphs and adults of *T. entomophagus* per litre. The distribution pattern was the same as that used with the predatory mites, so about 5 small clumps of the material fell on each plant.

Sampling of the populations of predators and pests was completed every week. Each time the total number of individuals (adults and nymphs) of predatory mites and thrips were counted on all the leaves of 5 randomly selected plants per treatment.

### Results

The densities of populations of *T. montodorensis* in the two treatments are given in Table 3, showing that in the presence of the additional food source (the *T. entomophagus* prey) the density of the population of *T. montdorensis* was much higher than in the control plot, which was without the additional food supply. By week four, just three weeks after the first introduction, the predators in the plot with an added food source was double that in the control plot. This difference between the two experimental units continued to increase every week, until by week six the population of predatory mites with the added food source was six times larger than that in the control. In contrast, the control plot population showed no further increase after the first small increase by week three. In the period when the trial was conducted the pressure of the main pest, the thrips, was low, and the populations counted of trips were always lower than two individuals per plant in both plots.

**Table 3. Total number of nymphs and adults of T. montdorensis counted in the leaves of five plants in each sampling date in each treatment.**

| Without providing in-crop food source (control) | | | |
|---|---|---|---|
| Week | MonControl, L | *T. entomophagus* | Number of individuals of *T. montdorensis* |
| 1 | 10 | 0 | - |
| 2 | 10 | 0 | 3 |
| 3 | 10 | 0 | 13 |
| 4 | 10 | 0 | 13 |
| 5 | 10 | 0 | 6 |
| 6 | 10 | 0 | 17 |
| 7 | 10 | 0 | 15 |

| Providing in-crop food source | | | |
|---|---|---|---|
| Week | MonControl, L | *T. entomophagus* | Number of individuals of *T. montdorensis* |
| 1 | 10 | 10 | - |
| 2 | 10 | 10 | 5 |
| 3 | 10 | 10 | 11 |
| 4 | 10 | 10 | 27 |
| 5 | 10 | 10 | 38 |
| 6 | 10 | 10 | 58 |
| 7 | 10 | 10 | 83 |

These results would guarantee a much better control of the pest when large infestations of thrips invaded. The result is very encouraging since a serious problem for commercial Chrysanthemum growers of cut-flower crops is that a single bite by a thrip into a petal when it is just developing in the un-opened bud will, when the flower is mature and fully open, show up as a distinct scar on the petal, quite visible to the naked eye.

Thus a small population of thrips, which will have no consequence to a vegetable grower, will be serious for the Chrysanthemum grower in that just one or two scars on a flower head will automatically cause it to be down-graded in its sales classification. Further, it will increase the effectiveness of biocontrol systems, and so reduce the high number of chemical applications that are used nowadays in this crop, which are not effective anymore due to resistances of the pest to the few permitted active ingredients. The new technique, described above, of giving an added food supply to the predator as it arrives on the crop, resulting in the quick development of a large predator density, will be of a significant monetary benefit to Chrysanthemum growers.

### Conclusions

- The introduction of prey mites at the top of the plants, applying a total of 3 releases, offers a very good solution to the problem of low development of the predatory mites during winter on cucumber crops.
- The prey mites can stay alive for at least 3 to 4 weeks on the plants, in winter conditions, remaining during this time as an alternative source of food for the predators.
- The populations of predatory mites increased up to 4 - 5 times compared to standard releases of the natural enemy when complementary food is not added.
- It is the first time in 8 years of experience advising on-crop protection in commercial cucumber crops that the team of technicians at Agrobio has counted such high numbers of eggs, nymphs and adults of predatory mites per leaf during winter.
- This system should guarantee a control of the current problem in which high infestations of thrips occur at the end of winter.
- A solution has been found for the problem of predator starvation in the absence of its pest prey, a common problem in cucumber crops, as well as other vegetable crops, ornamental crops and orchards.
Consideration of the other major alternative food sources given above show that whilst supplementary materials aimed at providing a source of sustainable food for the predators in the absence of the pest are known, and to a certain level are partially effective, none provide the absolute solution as to how to deal with the 'pre-pest' problem, namely the most vulnerable gap which occurs between the initial introduction of the biological agent and the arrival of the first pests.
The above experiments show that providing a sustainable food source for the predators, preferably the host on which the predators have been reared, provides a solution for the problem of keeping a predator population alive even if applied to a crop many weeks prior to pest invasion. It clearly demonstrates that the present invention provides a simple workable cost-effective method which is most advantageous to the success of biological control programs operating in protected and semi-protected crops.
After application to the crop, predator eggs can be expected to be laid on the same day the gravid female reaches a green leaf. The inventive step of this application ensures that such pregnant females come face to face with a food supply of their normal diet so that they will develop instead of declining. As shown by the experiments, the speed of development of the predator population can be remarkable. Thus, the present invention closes a long standing gap in the biological control defenses employed in the protected and semi- protected crop industry.

## Claims

1. A method for rearing and/or increasing the activity of at least one population of a predator mite species of the family Phytoseiidae comprising the steps:
providing at least one rearing population of a predator mite species of the family Phytoseiidae, and a first food source for this predator mite species, wherein the rearing population of the predator mite species and the first food source are both located in a common housing, which has at least one opening which allows individuals of the predator mite species to leave the common housing,
providing a second food source for the predator mite species, wherein the second food source comprises at least one population of a mite species belonging to the Astigmata, arranging the common housing in or on a plant, and
placing the second food source in or on the same or a different plant outside the common housing.

2. Method according claim 1,
**characterized in that**
the second food source is placed on a leaf of a plant, preferably on the top of a leaf, preferably on a leaf which is prone to be infested by a pest itself or which is in a distance of less than 50 cm, preferably less than 30 cm, preferably less than 15 cm to a part of the plant which is prone to be infested by a pest.

3. Method according to one of the preceding claims,
**characterized in that**
the common housing and the second food source are located in the same crop.

4. Method according to one of the preceding claims,
**characterized in that**
the second food source comprises a breeding population of a mite species belonging to the Astigmata and a third food source, wherein the third food source is a food for the mite species belonging to the Astigmata.

5. Method according to one of the preceding claims,
**characterized in that**
a plurality of common housings and a plurality of the second food sources are located in the crop.

6. Method according to one of the preceding claims,
**characterized in that**
each common housing comprises between 1 and 5000 individuals of the predatory mites

7. Method according to one of the preceding claims,
**characterized in that**
the density of the individuals of the mite species belonging to the Astigmata in the second food source is between 500.000 and 50 million individuals/liter.

8. Method according to one of the preceding claims,
**characterized in that**
a volume between 0,01 and 50 ml of the second food source is placed on one spot.

9. Method according to one of the preceding claims,
**characterized in that**
the mite species belonging to the Astigmata is selected from a group of mite species which is not a pest for the plant and/or crop in which the second food source is placed.

10. Method according to one of the preceding claims,
**characterized in that**
at least one of the mite species belonging to the Astigmata is *Carpoglyphus lactis* or *Tyrolichus casei* or *Thyreophagus entomophagus* or *Lepidoglyphus destructor.*

11. Method according to one of the preceding claims,
**characterized in that**
at least one of the mite species belonging to the Phytoseiidae is *Amblyseius swirskii* or *Typhlodromips (*=*Amblyseius) montdorensis* or *Neoseiulus californicus* or *Amblyseius andersoni* or *Neoseiulus cucumeris* or *Typhlodromalus (=Amblyseius) limonicus.*

## Patentansprüche

1. Verfahren zum Züchten und/oder Erhöhen der Aktivität von mindestens einer Population einer Raubmilbenart der Familie Phytoseiidae, umfassend die Schritte:
Bereitstellen mindestens einer Zuchtpopulation einer Raubmilbenart der Familie Phytoseiidae und einer ersten Nahrungsquelle für diese Raubmilbenart, wobei die Zuchtpopulation der Raubmilbenart und die erste Nahrungsquelle beide in einem gemeinsamen Gehäuse untergebracht sind, welches mindestens eine Öffnung aufweist, welche es den Individuen der Raubmilbenart ermöglicht, das gemeinsame Gehäuse zu verlassen,
Bereitstellen einer zweiten Nahrungsquelle für die Raubmilbenart, wobei die zweite Nahrungsquelle mindestens eine zu den Astigmata gehörende Population einer Milbenart umfasst,
Anordnen des gemeinsamen Gehäuses in oder auf einer Pflanze, und
Anordnen der zweiten Nahrungsquelle in oder auf der gleichen oder einer anderen Pflanze außerhalb des gemeinsamen Gehäuses.

2. Verfahren nach Anspruch 1,
**dadurch gekennzeichnet, dass**
die zweite Nahrungsquelle auf einem Blatt einer Pflanze angeordnet ist, vorzugsweise auf der Oberseite eines Blatts, vorzugsweise auf einem Blatt, welches selbst anfällig für eine Infektion durch einen Schädling ist oder welches in einem Abstand von weniger als 50 cm, vorzugsweise weniger als 30 cm, vorzugsweise weniger als 15 cm zu einem Teil der Pflanze ist, welches anfällig für eine Infektion durch einen Schädling ist.

3. Verfahren nach einem der vorhergehenden Ansprüche,
**dadurch gekennzeichnet, dass**
das gemeinsame Gehäuse und die zweite Nahrungsquelle in der gleichen Kultur angeordnet sind.

4. Verfahren nach einem der vorhergehenden Ansprüche,
**dadurch gekennzeichnet, dass**
die zweite Nahrungsquelle eine fortpflanzende Population einer zu den Astigmata gehörenden Milbenart und eine dritte Nahrungsquelle umfasst, wobei die dritte Nahrungsquelle Nahrung für die zu den Astigmata gehörende Milbenart ist.

5. Verfahren nach einem der vorhergehenden Ansprüche,
**dadurch gekennzeichnet, dass**
eine Vielzahl von gemeinsamen Gehäusen und eine Vielzahl von den zweiten Nahrungsquellen in der Kultur angeordnet sind.

6. Verfahren nach einem der vorhergehenden Ansprüche,
**dadurch gekennzeichnet, dass**
jedes gemeinsame Gehäuse zwischen 1 und 5000 Individuen der Raubmilben umfasst.

7. Verfahren nach einem der vorhergehenden Ansprüche,
**dadurch gekennzeichnet, dass**
die Individuendichte der zu den Astigmata gehörenden Milbenart in der zweiten Nahrungsquelle zwischen 500 000 und 50 Millionen Individuen/Liter liegt.

8. Verfahren nach einem der vorhergehenden Ansprüche,
**dadurch gekennzeichnet, dass**
ein Volumen zwischen 0,01 und 50 ml der zweiten Nahrungsquelle an einer Stelle angeordnet ist.

9. Verfahren nach einem der vorhergehenden Ansprüche,
**dadurch gekennzeichnet, dass**
die zu den Astigmata gehörende Milbenart ausgewählt ist aus einer Gruppe von Milbenarten, welche kein Schädling für die Pflanze und/oder Kultur ist, in welcher die zweite Nahrungsquelle angeordnet ist.

10. Verfahren nach einem der vorhergehenden Ansprüche,
**dadurch gekennzeichnet, dass**
mindestens eine der zu den Astigmata gehörenden Milbenarten *Carpoglyphus lactis* oder *Tyrolichus casei* oder *Thyreophagus entomophagus* oder *Lepidoglyphus destructor* ist.

11. Verfahren nach einem der vorhergehenden Ansprüche,
**dadurch gekennzeichnet, dass**
mindestens eine der zu den Phytoseiidae gehörenden Milbenarten *Amblyseius swirskii* oder *Typhlodromips (*= *Amblyseius) montdorensis* oder *Neoseiulus californicus* oder *Amblyseius andersoni* oder *Neoseiulus cucumeris* oder *Typhlodromalus (= Amblyseius) limonicus* ist.

## Revendications

1. Procédé pour élever et/ou accroître l'activité d'au moins une population d'une espèce d'acarien prédateur de la famille des Phytoseiidae, comprenant les étapes :
disposer au moins une population d'élevage d'une espèce d'acarien prédateur de la famille des Phytoseiidae, et une première source alimentaire pour cette espèce d'acarien prédateur, la population d'élevage d'espèce d'acarien prédateur et la première source alimentaire étant toutes deux situées dans un logement commun, lequel a au moins une ouverture qui permet aux individus de l'espèce d'acarien prédateurs de quitter le logement commun,
disposer une deuxième source alimentaire pour l'espèce d'acarien prédateur, la deuxième source alimentaire comprenant au moins une population d'une espèce d'acarien appartenant à l'espèce Astigmata,
agencer le logement commun dans ou sur une plante, et
placer la deuxième source alimentaire dans ou sur la même plante ou une plante différente, à l'extérieur du logement commun.

2. Procédé selon la revendication 1,
**caractérisé par le fait que**
la deuxième source alimentaire est placée sur une feuille d'une plante, de préférence sur le dessus d'une feuille, de préférence sur une feuille qui est susceptible d'être infestée elle-même par un organisme nuisible ou qui se trouve à une distance de moins de 50 cm, de préférence de moins de 30 cm, de préférence de moins de 15 cm, d'une partie de la plante qui est susceptible d'être infestée par un organisme nuisible.

3. Procédé selon l'une quelconque des revendications précédentes,
**caractérisé par le fait que**
le logement commun et la deuxième source alimentaire sont situés dans la même culture.

4. Procédé selon l'une quelconque des revendications précédentes,
**caractérisé par le fait que**
la deuxième source alimentaire comprend une population de reproduction d'une espèce d'acarien appartenant à l'espèce Astigmata et une troisième source alimentaire, la troisième source alimentaire étant un aliment pour l'espèce d'acarien appartenant à l'espèce Astigmata.

5. Procédé selon l'une quelconque des revendications précédentes,
**caractérisé par le fait qu'**
une pluralité de logements communs et une pluralité de deuxièmes sources alimentaires sont situés dans la culture.

6. Procédé selon l'une quelconque des revendications précédentes,
**caractérisé par le fait que**
chaque logement commun comprend entre 1 et 5000 individus des acariens prédateurs.

7. Procédé selon l'une quelconque des revendications précédentes,
**caractérisé par le fait que**
la densité des individus de l'espèce d'acarien appartenant à l'espèce Astigmata dans la deuxième source alimentaire est comprise entre 500 000 et 50 millions d'individus/litre.

8. Procédé selon l'une quelconque des revendications précédentes,
**caractérisé par le fait qu'**
un volume compris entre 0,01 et 50 ml de la deuxième source alimentaire est placé sur un point.

9. Procédé selon l'une quelconque des revendications précédentes,
**caractérisé par le fait que**
l'espèce d'acarien appartenant à l'espèce Astigmata est choisie dans un groupe d'espèces d'acariens qui n'est pas un organisme nuisible pour la plante et/ou culture dans laquelle la deuxième source alimentaire est placée.

10. Procédé selon l'une quelconque des revendications précédentes,
**caractérisé par le fait qu'**
au moins une des espèces d'acariens appartenant à l'espèce Astigmata est *Carpoglyphus lactis* ou *Tyrolichus casei* ou *Thyreophagus entomophagus* ou *Lepidoglyphus destructor.*

11. Procédé selon l'une quelconque des revendications précédentes,
**caractérisé par le fait qu'**
au moins une des espèces d'acariens appartenant aux Phytoseiidae est *Amblyseius swirskii* ou *Typhlodromips (=Amblyseius) montdorensis* ou *Neoseiulus californicus* ou *Amblyseius andersoni* ou *Neoseiulus cucumeris* ou *Typhlodromalus* (=*Amblyseius*) *limonicus.*
